# EUROPEAN PATENT APPLICATION

(11) **EP 3 842 533 A1**
(43) Date of publication of application: **30.06.2021**
(21) Application number: 19851437.4
(22) Date of filing: 22.08.2019
(51) Int. Cl.: C12N 15/10, G01N 35/00

(54) **METHOD FOR PATHOGEN ENRICHMENT AND NUCLEIC ACID EXTRACTION USING DEVICE FOR POINT-OF-CARE TESTING**

(30) Priority: 22.08.2018 KR 20180098022
(71) Applicant: INFUSION TECH, Anyang-si, Gyeonggi-do 14059 (KR)
(72) Inventor: SHIN, Yong, Seoul 05505 (KR); LEE, Eun Yeong, Guri-si Gyeonggi-do 11924 (KR); ZHAO, Fei, Seoul 02444 (KR)
(74) Representative: Roos, Peter
(86) International application number: PCT/KR2019/010720
(87) International publication number: WO 2020/040577

(57) **Abstract**

The present invention relates to a method of enriching pathogens and extracting nucleic acids using diatomaceous earth functionalized with amines and dimethyl suberimidate as a crosslinking agent, wherein negatively charged pathogens are electrically absorbed on the surface of positively charged diatomaceous earth by the crosslinking agent, and nucleic acid released after cell lysis can be immobilized and separated on the diatomaceous earth through reversible crosslinking with amine groups of diatomaceous earth. The above method can be used as a point-of-care testing because it is possible to concentrate pathogens and extract nucleic acids at the same time as an all-in-one device without the use of special equipment and electricity (electric power), and it is superior in time and cost savings than the conventional method, and the extracted nucleic acid has an advantage that can be usefully used for diagnosis and treatment of the diseases.

<110>	University of Ulsan Foundation For Industry Cooperation
	THE ASAN FOUNDATION

<120>	Method for pathogen enrichment and nucleic acid extract using
	device for point of care testing

<130>	AOP-2019-0059

<160>	4

<170>	KoPatentIn 3.0

<210>	1
<211>	20
<212>	DNA
<213>	Artificial Sequence

<220>
<223>	IS711 forward


<400>	1
gcttgaagct tgcggacagt                                                   20


<210>	2
<211>	17
<212>	DNA
<213>	Artificial Sequence

<220>
<223>	IS711 reverse


<400>	2
ggcctaccgc tgcgaat                                                      17


<210>	3
<211>	19
<212>	DNA
<213>	Artificial Sequence

<220>
<223>	invA forward


<400>	3
tatcgccacg ttcgggcaa                                                    19


<210>	4
<211>	19
<212>	DNA
<213>	Artificial Sequence

<220>
<223>	invA reverse


<400>	4
tcgcaccgtc aaaggaacc                                                    19

## Description

### [Technical Field]

The present invention relates to a method of pathogen enrichment and nucleic acid extraction using a device for point-of-care testing.

### [Background Art]

Pathogen diagnosis is very important in global health problems, especially in resource-constrained environments, such as centralized laboratory technology. To date, various methods such as cell culture, blood chemistry, flow cytometry, immunoassay and nucleic acid testing (NAT) have been developed for the diagnosis of pathogens, and the cell culture is widely used for clinical diagnosis, but it has the disadvantages of requiring time-consuming procedures, high cost, species-specific protocols, laboratory environment and equipment. On the other hand, nucleic acid testing is gaining interest because it has a relatively fast and universal protocol as well as applications for diagnostic tests such as window period diagnosis, immune mutant virus detection and immunosilent/occult infection identification. However, the conventional nucleic acid test-based analysis has a disadvantage that both commercially available products and laboratory-developed assays are generally complex. Thus, complex pretreatments that necessitate laboratory-based work, such as skilled technicians, specific equipment and multiple steps, are required. These demands limit the use of nucleic acid testing in point of care testing (POCT).

A point of care testing based on nucleic acid testing involve three main aspects of sample preparation, template amplification and signal detection. Researches focused on simple and effective amplification and detection as a method to improve the point of care testing based on nucleic acid testing have been conducted, but studies focused on the initial sample preparation stage have also been conducted. A nucleic acid-based point of care testing has been developed that can be amplified directly from a sample and does not require much extraction, but it is still limited due to low sensitivity and high cost. Sample preparation, including extraction of nucleic acids from biological substrates and removal of chemical inhibitors, is very important because high quality nucleic acids are the basis of all sub-analysis. In particular, this step should be tailored for open environments rather than laboratory conditions to remove the boundary to resource-constrained environments from aseptic laboratories.

Unlike developed countries, mass centralized laboratory-based diagnostics are not suitable for resource-constrained environments due to the limitations of high cost of permanently integrated facilities and the skilled technicians. Therefore, disposable point-of-care assays can be a solution to resource-constrained environments. For the past 10 years, immuno-chromatographic strip (ICS) testing has been one of the diagnostic assays that have been successfully used in resource-constrained environments, but it has a drawback that is limited to deep sample analysis.

For point-of-care diagnostic use, the analysis should be simplified (i.e. there are few manufacturing and application processes, facilities or training requirements), and should be fast, reliable (logistics and storage aspects) and optimized for low cost. In particular, it is essential to apply this assay to common clinical samples such as urine, blood and sputum. However, this is difficult due to PCR inhibitors that are widely stimulated in biological samples as well as pathogen extraction from samples. Thus, an applicable solution for the integration of pathogen enrichment and nucleic acid sample preparation can contribute to both clinical detection and point-of-care analysis. In addition, in low-developed countries including Africa, the use of laboratory-based equipment is limited due to the difficulty of supplying electricity and therefore, point-of-care analysis capable of enriching pathogens and extracting nucleic acids without the use of electricity (electric power) has the advantage of being widely used in resource-limited environments.

### [Disclosure]

### [Technical Problem]

An object of the present invention is to provide a pathogen enrichment and nucleic acid extraction device for point-of-care testing.

Another object of the present invention is to provide a pathogen enrichment and nucleic acid extraction method using the above device.

### [Technical Solution]

The present invention provides a pathogen enrichment and nucleic acid extraction device for point-of-care testing comprising: an inlet; a reaction unit connected to the inlet and mixing a sample containing a pathogen introduced through the inlet, a crosslinking agent and diatomaceous earth modified with a silane compound; a filter unit connected to the reaction unit and capable of blocking a sample containing a pathogen, a crosslinking agent and a diatomaceous earth modified with a silane compound and passing a substance having a size corresponding to a nucleic acid; and an outlet unit connected to the filter unit and separating nucleic acid.

Also, the present invention provides a pathogen enrichment and nucleic acid extraction method comprising: a first step of providing the pathogen enrichment and nucleic acid extraction device for point-of-care testing; a second step of introducing a sample containing a pathogen, a crosslinking agent, and diatomaceous earth modified with a silane compound through an inlet of the device; a third step of fixing the pathogen on a surface of the diatomaceous earth modified with a silane compound by a crosslinking agent in a reaction unit of the device; a fourth step of introducing a lysis buffer solution through the inlet of the device; a fifth step of fixing nucleic acid extracted from the pathogen to a surface of the diatomaceous earth modified with a silane compound in a reaction unit of the device; a sixth step of introducing an elution buffer through the inlet of the device; and a seventh step of separating the nucleic acid through a filter unit of the device and obtaining the nucleic acid through an outlet part.

### [Advantageous Effects]

The present invention relates to a method of enriching pathogens and extracting nucleic acids using diatomaceous earth functionalized with amines and dimethyl suberimidate as a crosslinking agent, wherein negatively charged pathogens are electrically absorbed on the surface of positively charged diatomaceous earth by the crosslinking agent, and nucleic acid released after cell lysis can be immobilized and separated on the diatomaceous earth through reversible crosslinking with amine groups of diatomaceous earth. The above method can be used as a point-of-care testing because it is possible to concentrate pathogens and extract nucleic acids at the same time as an all-in-one device without the use of special equipment and electricity (electric power), and it is superior in time and cost savings than the conventional method, and the extracted nucleic acid has an advantage that can be usefully used for diagnosis and treatment of the diseases.

### [Description of Drawings]

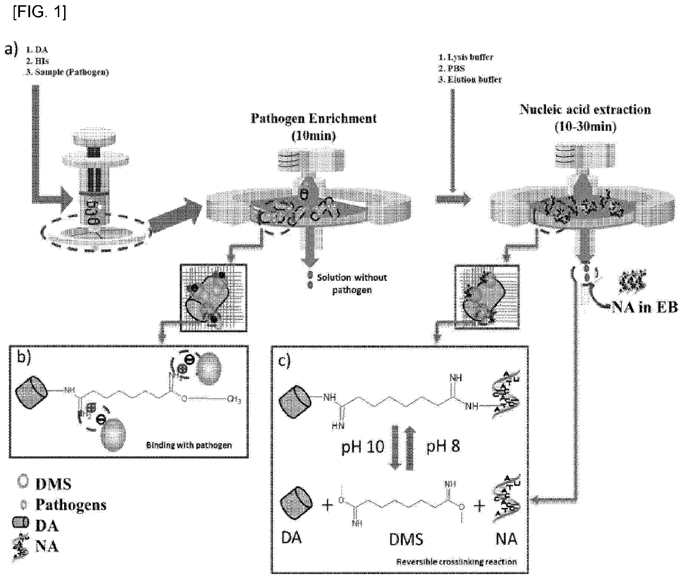

FIG. 1 is a diagram showing a method of pathogen enrichment and nucleic acid extraction by a portable syringe filter device using diatomaceous earth functionalized with amine (DA) and dimethyl suberimidate (DMS), and (a) mixing DA, pathogen and DMS in the syringe; (b) pathogen concentration process in which the pathogen is absorbed on the DA surface by DMS and subsequently blocked by a filter; and (c) nucleic acid isolation through reversible crosslinking reaction between the amine group of DA and the nucleic acid released from the dissolved pathogen.

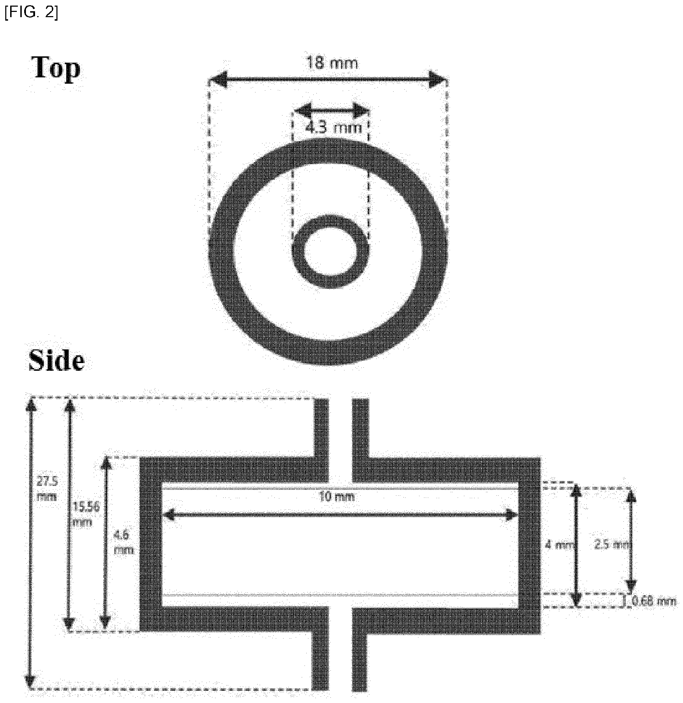

FIG. 2 is a view showing the top and side surfaces of the syringe filter.

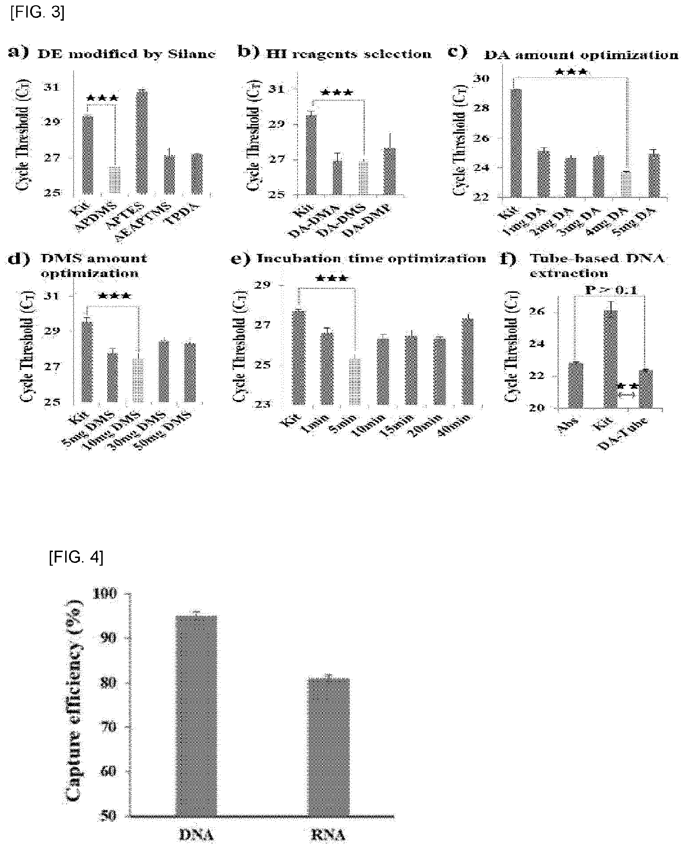

FIG. 3 shows the results of real-time quantitative PCR for optimizing the nucleic acid separation process, and (a) optimization of a silane compound for DA production; (b) optimization of isotype 2-functional imidoester; (c) optimization of DA concentration; (d) optimization of DMS concentration; (e) optimization of incubation time for RNA isolation; and (f) evaluation of the DNA template isolated under the optimized conditions shown as CT values.
FIG. 4 shows the capture efficiency of DNA and RNA using an optimized nucleic acid separation process.

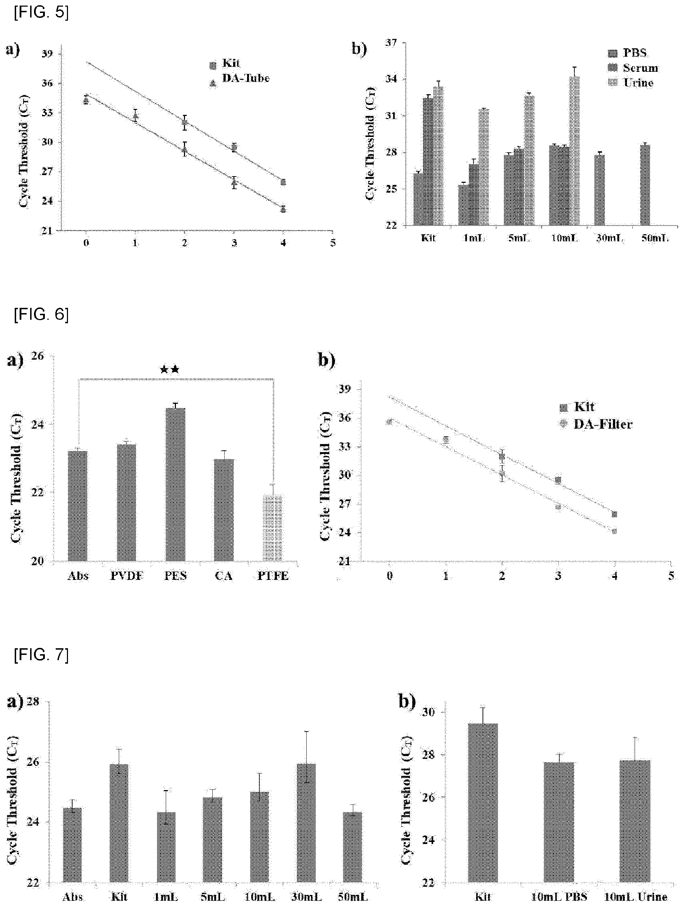

FIG. 5 shows the results of real-time quantitative PCR for evaluating the DA-DMS tube system, and (a) evaluation of RNA templates isolated from pathogen samples using the DA-DMS tube system and commercial kit system; and (b) evaluation of the RNA template separated from various samples and the volume shown as a CT value.
FIG. 6 shows the results of real-time quantitative PCR for evaluating the DA-DMS filter system, and (a) evaluation of the syringe filter type; and (b) evaluation of RNA isolated from pathogen samples using the DA-DMS filter system and a commercial kit system shown as a C_{T} value.
FIG. 7 shows the results of real-time quantitative PCR for evaluating the processing capacity of a large sample of the DA-DMS filter system, and (a) evaluation of RNA templates isolated from samples of various volumes; and (b) evaluation of RNA templates isolated from different samples shown as a C_{T} value.

### [Best Mode]

The inventors of the present invention provide a method of pathogen enrichment and nucleic acid extraction that can be easily integrated into other assays and the method is an integrated device designed to simplify the entire process and can be used for point-of-care testing. Pathogens can be concentrated by a syringe filter in the presence of amine-functionalized diatomaceous earth (DA) and dimethyl suberimidate (DMS), and nucleic acid extraction is possible without additional equipment.

Positively charged diatomaceous earth can be electrically bound to negatively charged pathogens in an aqueous environment. However, the presence of dimethyl suberimidate, which can bind to the amine groups of diatomaceous earth, can lead to the formation of more positive charges, thereby intensifying the pathogen enrichment process. Therefore, negatively charged pathogens can be directly absorbed to diatomaceous earth in a 1 mL sample or directly through a short incubation in a large sample (50 mL). Meanwhile, dimethyl suberimidate is known as a crosslinking agent for the reversible reaction of the amine group of a nucleic acid or protein. Since the reaction can be reversed to a controlled pH, extraction of a nucleic acid sample used for diagnosis can be easily achieved by injection of other buffers, and in particular, the diagnostic method of the present invention has an advantage suitable for various clinical samples.

FIG. 1 shows a schematic diagram of the integrated analysis method of the present invention, and the portable syringe filter device can effectively concentrate pathogens and separate nucleic acids and it has the advantage that the analysis production and manipulation procedure is simple, the cost is low, and the total working time can be reduced to at least 20 minutes. In addition, it does not require laboratory facilities and can provide a universal protocol for a variety of clinical samples.

Thus, the present invention a pathogen enrichment and nucleic acid extraction device for point-of-care testing comprising: an inlet; a reaction unit connected to the inlet and mixing a sample containing a pathogen introduced through the inlet, a crosslinking agent and diatomaceous earth modified with a silane compound; a filter unit connected to the reaction unit and capable of blocking a sample containing a pathogen, a crosslinking agent and a diatomaceous earth modified with a silane compound and passing a substance having a size corresponding to a nucleic acid; and an outlet unit connected to the filter unit and separating nucleic acid.

The filter may have pores having an average diameter of 0.5 to 1 µm, and may have a diameter in the range of 10 to 30 mm and a thickness in the range of 1 to 10 mm, but it is not limited thereto.

The filter may be selected from the group consisting of nano- and micro-filtration membrane filters, reverse osmosis filters, hollow fiber membrane filters, and ultrafiltration membrane filters, but it is not limited thereto.

In addition, the present invention provides a pathogen enrichment and nucleic acid extraction method comprising: a first step of providing a pathogen enrichment and nucleic acid extraction device for point-of-care testing; a second step of introducing a sample containing a pathogen, a crosslinking agent, and diatomaceous earth modified with a silane compound through an inlet of the device; a third step of fixing the pathogen on a surface of the diatomaceous earth modified with a silane compound by a crosslinking agent in a reaction unit of the device; a fourth step of introducing a lysis buffer solution through the inlet of the device; a fifth step of fixing nucleic acid extracted from the pathogen to a surface of the diatomaceous earth modified with a silane compound in a reaction unit of the device; a sixth step of introducing an elution buffer through the inlet of the device; and a seventh step of separating the nucleic acid through a filter unit of the device and obtaining the nucleic acid through an outlet part.

Samples containing the pathogen of the second step may be any one selected from the group consisting of feces, urine, tears, saliva, external secretions from skin, external secretions from respiratory tract, external secretions from intestinal tract, external secretions from digestive tract, plasma, serum, blood, spinal fluid, lymph fluid, body fluids and tissues of object suspected of being infected with the pathogen, but it is not limited thereto.

The pathogen of the second step may be a microorganism, and the microorganism may be virus, bacteria, fungi, protozoa, *Rickettsia* or spirochaete, but it is not limited thereto.

The crosslinking agent of the second step may be selected from the group consisting of dimethyl suberimidate (DMS), dimethyl adipimidate (DMA), dimethyl pimelimidate (DMP) and dimethyl 3,3'-dithiobispropionimidate (DTBP), but it is not limited thereto.

The silane compound of the second step may be any one selected from the group consisting of 3-aminopropyl(diethoxy)methylsilane (APDMS), (3-aminopropyl)triethoxysilane (APTES), (3-aminopropyl)trimethoxysilane, (1-aminomethyl)triethoxysilane, (2-aminoethyl)triethoxysilane, (4-aminobutyl)triethoxysilane, (5-aminopentyl)triethoxysilane, (6-aminohexyl)triethoxysilane, N-[3-(trimethoxysilyl)propyl]ethylenediamine, N-[3-(trimethoxysilyl)propyl]diethylenetriamine, [3-(2-aminoethylamino)propyl]trimethoxysilane (AEAPTMS) and 3-[(trimethoxysilyl)propyl]diethylenetriamine (TMPTA), but it is not limited thereto.

In the third step, a negatively charged pathogen may be immobilized by an electrostatic bond to the surface of diatomaceous earth modified with a positively charged silane compound by a crosslinking agent.

In the fifth step, the nucleic acid extracted from the pathogen may be immobilized on the surface of the diatomaceous earth through reversible crosslinking with the amine group of the diatomaceous earth modified with a silane compound.

The pathogen enrichment and nucleic acid extraction may be completed within 15 to 30 minutes.

The nucleic acid may be selected from the group consisting of DNA, RNA, circulating tumor DNA (ctDNA) and cell free DNA (cfDNA, but it is not limited thereto.

The cfDNA is a circulating free nucleic acid and refers to DNA circulating in the blood. The circulating free nucleic acid specifically includes circulating free DNA, circulating free RNA, and the like, and preferably may be circulating free DNA, but it is not limited thereto. T8he circulating free nucleic acid generally exhibits a length of 1000 bp or less (DNA), 100 nt or less (RNA) in plasma or serum, but it is not limited thereto.

The ctDNA refers to DNA that is isolated from cancer cells and circulates in blood. It has both tumor-specific mutations and epigenetic mutations and accounts for a very small amount of total circulating DNA in the blood. The size of circulating tumor DNA varies from 50 bp to 250 bp, but it is not limited thereto.

Hereinafter, examples will be described in detail to aid understanding of the present invention. However, the following examples are only intended to illustrate the present invention and the scope of the present invention is not limited to the following examples. The examples of the present invention are provided to more completely explain the present invention to those skilled in the art.

### Example 1: Materials

Diatomaceous earth (DE), (3-aminopropyl) triethoxysilane (APTES, 99%), 3-aminopropyl(diethoxy)methylsilane (APDMS, 97%), 3-(2-aminoethylamino)propyl]trimethoxysilane (AEAPTMS, 80%) and N1-(3-trimethoxysilylpropyl)diethylenetriamine (TPDA) were purchased from Sigma Aldrich.

### Example 2: Preparation of diatomaceous earth functionalized with amine

Diatomaceous earth (DE) was washed by vigorous stirring for 30 minutes with distilled water (DW), and then centrifuged to remove sediments containing impurities. An amine-functionalized DE (DA) was used as a concentration and extraction matrix, and the surface of the washed DE was synthesized by reacting with an amine group of a silane compound. The DA was prepared as follows. Briefly, 5 mL of silane was added dropwise to an ethanol mixture containing 100 mL of 5% (v/v) distilled water and acidified with acetic acid (pH 5). 2 g of washed DE was added under vigorous stirring and reacted at room temperature for 4 hours. DA was washed with ethanol and then vacuum dried overnight and stored until used for further analysis.

### Example 3: Cell culture

Brucella ovis (ATCC 25840) was used to evaluate the diagnosis of pathogenicity. Brucella obis was cultured in Brucella agar (Sigma-Aldrich) at 37°C and 5% CO₂ using 5% defibrinated sheep blood (MBcell, Korea). Salmonella enterica (ATCC 14028) was inoculated in trypticase soy broth (TSB, BD Difco) and incubated for 24 hours at 37°C in an aerobic atmosphere. After incubation, the bacterial suspension was quantified by the agar plate method, and then diluted to different concentrations using phosphate buffered saline (PBS).

### Example 4: Pathogen enrichment-tube basis

DA and dimethyl suberimidate (DMS, Sigma-Aldrich) were selected as the concentration and extraction matrix according to the optimized process. First, 120 µL of APDMS-DE suspension (50 mg/mL in DW) and 100 µL of DMS solution (200 mg/mL in DW) were added to the sample. Pathogens were attached to the surface of APDMS-DE based on the reversible crosslinking reaction through a homobifunctional immidoester (HI) group by reacting for 1 to 30 minutes at room temperature using a 99 rpm rotary mixer (Topscien Instrument Co., Ltd., Ningbo, China). The pathogen-absorbed APDMS-DE was collected by centrifuging at 1000 rpm for 1 minute to remove the supernatant. Thereafter, the mixture was washed with 1 mL of phosphate buffered saline and centrifuged once more to remove the supernatant and 100 µL of a sample containing the pathogen was obtained.

### Example 5: Nucleic acid extraction-tube basis

To isolate the nucleic acid from the concentrated sample, 20 µL of proteinase K, 150 µL of self-lysis buffer (100 mM Tris-HCI (pH 8.0), 10 mM ethylenediaminetetraacetic acid, 1% sodium dodecyl sulfate and 10% Triton X-100), 30 µL of lysozyme solution (30 mg/mL in DW, Sigma-Aldrich) and 10 µL of RNase-Free DNase solution (for RNA, Qiagen) were added to the sample. Thereafter, the supernatant was removed by incubation at 56°C for 30 minutes (for DNA) or 10 minutes (for RNA) at room temperature using a stirrer at 850 rpm, followed by centrifugation. APDMS-DE to which nucleic acid was bound was washed twice with 200 µL of phosphate buffered saline, and all solutions were mixed by gently pipetting. Then, 100 µL of elution buffer (EB) (10 mM sodium bicarbonate, pH> 10, adjusted with NaOH, Sigma-Aldrich) was added to the solution, incubated for 1 minute at room temperature, and then centrifuged and the supernatant containing nucleic acids was transferred to a 1.5 mL tube and stored at -20°C. In addition, the commercial kits (QIAamp DNA Mini Kit and QNagen RNeasy Mini Kit) were used as sample of a positive control and nucleic acids was extracted according to the recommended protocol.

### Example 6: Pathogen enrichment-filter basis

A schematic diagram of the pathogen enrichment procedure using a syringe filter and DA-HI system was shown in FIG. 1B. 120 µL of APDMS-DE suspension (50 mg/mL in DW) and 100 µL of DMS solution (200 mg/mL in DW) were added to the sample. Pathogens were attached to the surface of APDMS-DE based on the reversible cross-crosslinking reaction through the HI group by reacting for 1 to 30 minutes at room temperature using a 99 rpm rotary mixer (Topscien Instrument Co., Ltd., Ningbo, China). The pathogen-absorbed APDMS-DE mixture was passed through a polytetrafluoroethylene (PTFE) syringe filter (Whatman, USA) having a void of 1.0 µm. Then, 1 mL of phosphate buffered saline was passed to wash the filter in which the pathogen was captured. Finally, the plunger of the syringe injected with air was strongly pressed to remove the solution from the filter.

### Example 7: Nucleic acid extraction-filter basis

In order to isolate the nucleic acid from the concentrated sample, 20 µL of proteinase K, 150 µL of self-lysis buffer, 30 µL of lysozyme solution and 10 µL of RNase-Free DNase solution were injected into the syringe filter. Next, the syringe filter was lightly vortexed, incubated at 56°C (for DNA) or at room temperature (for RNA), and then the filter in which the nucleic acid was captured was washed by passing the filter twice with 1 mL of phosphate buffered saline. The plunger of the syringe injected with air was strongly pressed to remove the solution from the filter, and 100 µL of elution buffer was injected into the filter, followed by reaction at room temperature for 1 minute. Finally, the solution containing the nucleic acid was eluted by strongly pressing the plunger of the syringe injected with air and stored at -20°C.

### Example 8: Real-time PCR

To check the quality of the isolated DNA, PCR and real-time PCR were performed, and RT-PCR and real-time RT-PCR were performed to verify the quality of the extracted RNA. The primers used in the experiment are shown in Table 1 below.

**[Table 1]**

| Sample | Target | Sequence (5' -> 3') |
|---|---|---|
| B. ovis | IS711 | F: GCTTGAAGCTTGCGGACAGT |
| | | R: GGCCTACCGCTGCGAAT |
| S.enterica | invA | F: TATCGCCACGTTCGGGCAA |
| | | R:TCGCACCGTCAAAGGAACC |

### Experimental Example 1: Preparation of an all-in-one portable analysis device

The assay used to collect nucleic acid templates from various samples was based on a commercial PTFE syringe filter and was pumped with a syringe as shown in FIG. 1A. The use of DA and DMS improved pathogen concentration. DMS, an imidoester, is linked to the amine group of DA to create a positively charged imidine bond that can directly attract negatively charged pathogens. Moreover, DA imparted with a nanoporous structure exhibits a very strong absorption capacity and a very high reaction area, which contributes to improved performance. Syringe filters were used to isolate absorbed pathogens and DA. Therefore, the concentration process as shown in FIG. 1B can be easily performed by mixing DMS and DA with pathogens in a solution and then injecting them into a filter.

After washing, the pathogens in the filter were dissolved *in situ.* It is known that DNA and RNA are separated by a reversible crosslinking reaction. DMS acts as a crosslinking agent between the nucleic acid and the amine group of DA. That is, the nucleic acid is immobilized on the surface of DA through a crosslinking reaction under the reaction buffer pH, whereas the elution buffer at a high pH reverses the reaction and subsequently releases the captured nucleic acid. Therefore, the nucleic acid template can be easily extracted by adjusting only the pH of the injected buffer as shown in FIG. 1C. In particular, the assay of the present invention shows excellent efficacy in RNA isolation. Due to the strong covalent bond between RNA and DA, the captured RNA is stable enough to withstand degradation. The assay method of the present invention does not require additional equipment or pretreatment to remove RNase, which can be easily applied to a point-of-care system.

### Experimental Example 2: Optimization of all-in-one portable analysis method

Before incorporating the method, all relevant factors must be individually optimized. Accordingly, in FIG. 3, the extraction efficiency of DA and DMS conditions as well as incubation time in the tube was evaluated through a cycle threshold (CT) of quantitative RT-PCR (qRT-PCR). 1 mL of Brucella Obis sample (10⁴ CFU mL⁻¹ in PBS) was used for all optimization processes. Nucleic acids were extracted from the same sample using commercial kits (QIAamp DNA Mini Kit and QNagen RNeasy Mini Kit) as a reference. The amine functionalization of DA is in its infancy and contributes to the pathogen concentration and RNA extraction process.

Different silane compounds were also analyzed in FIG. 3A. The lower the C_{T}, the higher the quality of the nucleic acid template, so APDMS was finally selected. On the other hand, as a crosslinking agent, various imidoesters were analyzed in FIG. 3B, and DMS showed better performance than other imidoesters (DMA, DMP). In particular, only DMS, which is stable at room temperature, can be helpful for storage and transport of the assay. Thereafter, the concentrations of DA and DMS were optimized, respectively, as shown in FIG. 3C - FIG. 3D. This completes the optimization of pathogen concentration. After cell lysis, RNA is released and simultaneously binds to DA in DMS. In addition, the performance according to the incubation time was analyzed. As shown in FIG. 3E, the highest efficiency was exhibited at 5 minutes of incubation time. In addition, the optimized system was used to analyze DNA extraction as shown in FIG. 3F. The capture efficiency was evaluated by comparing the C_{T} values. A sample of 100 µL (Brucella obis in PBS, 10⁵ CFU mL⁻¹) containing the same concentration of the pathogen was separated into a kit and used as a reference. The capture efficiencies for RNA and DNA separation were 95% and 81%, respectively (FIG. 4). Thereafter, the optimized system was then integrated with the syringe filter. The capture efficiency was at least 85% compared to the reference. That is, the all-in-one portable analysis for pathogen concentration and nucleic acid extraction was optimized with the type of reagent (FIG. 3A - FIG. 3B), concentration (FIG. 3C - FIG. 3D) and incubation time (FIG. 3E).

### Experimental Example 3: Evaluation of all-in-one approach using tube

In order to confirm the point-of-care sample preparation ability using the system of the present invention, 1 mL (10° to 10⁴ CFU mL⁻¹) of the pathogen Brucella obis sample was used. Using the system of the present invention and a commercial kit, RNA templates were simultaneously extracted and qPCR analysis was performed for pathogen detection. The detection limit of the system of the present invention was observed as 10° CFU mL⁻¹ as shown in FIG. 5A, and was 100 times more sensitive than the kit system (10² CFU mL⁻¹). In addition, the analysis method of the present invention can process up to 50 mL of samples from various matrices such as PBS, urine, and serum as shown in FIG. 5B. As shown in FIG. 5B, 10³ CFU Brucella obis was added to different volumes of medium, and the RNA template extracted by the analysis method of the present invention in 10 mL diluted liquid showed very effective amplification. The templates from PBS and urine samples were delayed by 2.3 and 0.8 cycles, respectively. In particular, all templates of the serum sample were observed in the initial cycle, and 0because the serum sample contains much more RNase, so the released RNA is easily degraded by the usual method, whereas the RNA extracted in this assay shows strong resistance to RNase degradation due to the robust covalent bond. Therefore, the analysis method of the present invention can improve the limit of detection of pathogens as well as a large amount of low concentration samples.

### Experimental Example 4: Performance evaluation of all-in-one portable assay

In order to completely overcome the laboratory dependence, the method of the present invention was integrated with a syringe filter. Several commercial filters were analyzed under optimized conditions, and enrichment results according to various filter types (cellulose acetate (CA), polyethersulfone (PES), polyvinylidene fluoride (PVDF) and polytetrafluoroethylene (PTEE)) were shown in FIG. 6A. In particular, a fast C_{T} cycle was observed in the PTFE filter, which contributed to the highest alkali saturation and low protein binding rate. Next, RNA templates were prepared from pathogen samples of various concentrations using a syringe filter, and detection analysis was then performed by qPCR. As can be seen in FIG. 6b, the assay method of the present invention exhibited a very low detection limit with 10° CFU mL⁻¹, which is 100 times higher than that of the kit system (10² CFU mL⁻¹), similar to the protocol using a tube. Both the tube and kit system showed high efficiency in pathogen concentration and in situ nucleic acid extraction, which means that the protocol of the present invention can be easily integrated with other assays to extract high quality RNA templates in the field.

In addition, the analysis method of the present invention can easily process a large amount of samples with a simple loading step using a syringe. Samples of up to 50 mL were tested, and it was confirmed that enrichment and nucleic acid extraction were stably performed from samples of various volumes as shown in FIG. 7A. In addition, as shown in FIG. 7B, the analysis performance of the present invention was verified in other biological matrices, and it was confirmed that enrichment and nucleic acid extraction were excellent in urine samples.

While the present invention has been particularly described with reference to specific embodiments thereof, it is apparent that this specific description is only a preferred embodiment and that the scope of the present invention is not limited thereby to those skilled in the art. That is, the practical scope of the present invention is defined by the appended claims and their equivalents.

The scope of the present invention is indicated by the claims to be described later, and all changes or modified forms derived from the meaning and scope of the claims and their equivalent concepts should be interpreted as being included in the scope of the present invention.

## Claims

1. A pathogen enrichment and nucleic acid extraction device for point-of-care testing comprising:
an inlet;
a reaction unit connected to the inlet and mixing a sample containing a pathogen introduced through the inlet, a crosslinking agent and diatomaceous earth modified with a silane compound;
a filter unit connected to the reaction unit and capable of blocking a sample containing a pathogen, a crosslinking agent and a diatomaceous earth modified with a silane compound and passing a substance having a size corresponding to a nucleic acid; and
an outlet unit connected to the filter unit and separating nucleic acid.

2. The pathogen enrichment and nucleic acid extraction apparatus for point-of-care testing of claim 1, wherein the filter has pores having an average diameter of 0.5 to 1 µm and has a diameter of 10 to 30 mm and a thickness of 1 to 10 mm.

3. The pathogen enrichment and nucleic acid extraction device for point-of-care testing of claim 1, wherein the filter is selected from the group consisting of a nano- and micro-filtration membrane filter, a reverse osmosis filter, a hollow fiber membrane filter and an ultrafiltration membrane filter.

4. A pathogen enrichment and nucleic acid extraction method comprising;
a first step of providing a pathogen enrichment and nucleic acid extraction device for point-of-care testing according to any one of claims 1 to 3;
a second step of introducing a sample containing a pathogen, a crosslinking agent, and diatomaceous earth modified with a silane compound through an inlet of the device;
a third step of fixing the pathogen on a surface of the diatomaceous earth modified with a silane compound by a crosslinking agent in a reaction unit of the device;
a fourth step of introducing a lysis buffer solution through the inlet of the device;
a fifth step of fixing nucleic acid extracted from the pathogen to a surface of the diatomaceous earth modified with a silane compound in a reaction unit of the device;
a sixth step of introducing an elution buffer through the inlet of the device; and
a seventh step of separating the nucleic acid through a filter unit of the device and obtaining the nucleic acid through an outlet part.

5. The pathogen enrichment and nucleic acid extraction method of claim 4, wherein the sample containing the pathogen is any one selected from the group consisting of feces, urine, tears, saliva, external secretions from skin, external secretions from respiratory tract, external secretions from intestinal tract, external secretions from digestive tract, plasma, serum, blood, spinal fluid, lymph fluid, body fluids and tissues of object suspected of being infected with the pathogen.

6. The pathogen enrichment and nucleic acid extraction method of claim 4, wherein the pathogen in the second step is a microorganism.

7. The pathogen enrichment and nucleic acid extraction method of claim 6, wherein the microorganism is virus, bacteria, fungi, protozoa, *Rickettsia* or spirochaeta.

8. The pathogen enrichment and nucleic acid extraction method of claim 4, wherein the crosslinking agent in the second step is selected from the group consisting of dimethyl suberimidate (DMS), dimethyl adipimidate (DMA), dimethyl pimelimidate (DMP) and dimethyl 3,3'-dithiobispropionimidate (DTBP).

9. The pathogen enrichment and nucleic acid extraction method of claim 4, wherein the silane compound in the second step is any one selected from the group consisting of 3-aminopropyl(diethoxy)methylsilane (APDMS), (3-aminopropyl)triethoxysilane (APTES), (3-aminopropyl)trimethoxysilane, (1-aminomethyl)triethoxysilane, (2-aminoethyl)triethoxysilane, (4-aminobutyl)triethoxysilane, (5-aminopentyl)triethoxysilane, (6-aminohexyl)triethoxysilane, N-[3-(trimethoxysilyl)propyl]ethylenediamine, N-[3-(trimethoxysilyl)propyl]diethylenetriamine, [3-(2-aminoethylamino)propyl]trimethoxysilane (AEAPTMS) and 3-[(trimethoxysilyl)propyl]diethylenetriamine (TMPTA).

10. The pathogen enrichment and nucleic acid extraction method of claim 4, wherein in the third step, a negatively charged pathogen is immobilized by an electrostatic bond on the surface of diatomaceous earth modified with a positively charged silane compound by a crosslinking agent.

11. The pathogen enrichment and nucleic acid extraction method of claim 4, wherein in the fifth step, the nucleic acid extracted from the pathogen is immobilized on the surface of the diatomaceous earth through reversible crosslinking with amine group of the diatomaceous earth modified with a silane compound.

12. The pathogen enrichment and nucleic acid extraction method of claim 4, wherein the pathogen enrichment and nucleic acid extraction are completed within 15 to 30 minutes.

13. The pathogen enrichment and nucleic acid extraction method of claim 4, wherein the nucleic acid is selected from the group consisting of DNA, RNA, circulating tumor DNA (ctDNA) and cell free DNA (cfDNA).
